# EUROPEAN PATENT APPLICATION

(11) **EP 0 838 150 A1**
(43) Date of publication of application: **29.04.1998**
(21) Application number: 97908504.0
(22) Date of filing: 26.03.1997
(51) Int. Cl.: A01H 5/00, A01H 1/00

(54) **METHOD FOR TRANSFORMING ORCHIDACEOUS PLANT USING AGROBACTERIUM**

(30) Priority: 28.03.1996 JP 73962/96
(71) Applicant: NIPPON PAINT CO., LTD., Osaka-shi Osaka 530 (JP)
(72) Inventor: MIURA, Yasutaka, Takatsuki-shi, Osaka 569-11 (JP); YAMAMOTO, Yoshikazu, Neyagawa-shi, Osaka 572 (JP)
(74) Representative: Kolb, Helga, Dr. Dipl.-Chem.
(86) International application number: JP9701004
(87) International publication number: WO9735468

(57) **Abstract**

Provided is a method of transforming an orchidaceous plant comprising the step of infecting a tissue derived from said orchidaceous plant with *Agrobacterium* having a gene to be introduced into the plant. The present invention enables the transformation of an orchidaceous plant with *Agrobacterium* for the first time.

## Description

### TECHNICAL FIELD

The present invention relates to a method of generating transgenic orcidaceae plants, particularly, a method of transforming an orcidaceae plant with *Agrobacterium* containing a desired gene.

### BACKGROUND ART

Nowadays, in the field of plant biotechnology, there are some methods for plant transformation known in the art including protoplast methods, particle gun shooting, and *Agrobacterium* infection method and so on.

The protoplast method, either the electroporation or the polyethylene glycol method, is a method in which a desired DNA is introduced into the protoplast and a plant body is regenerated from said protoplast. However, there is a problem that this method cannot be applied if the plant species for which the system for regenerating plants from protoplast has not been established. In addition, the efficiency of introducing the gene by this method is somewhat low. The method of particle gun is a method in which the desired gene attached to fine metal particles is shot into cells at a high speed thereby carrying out the transformation. The method can be applied to principally any tissue or cell of the plant and provides high transformation efficiency. However, it requires a special and expensive apparatus, a particle gun.

On the other hand, a method of transforming plants with an infectious bacterium, *Agrobacterium*, is widely used for dicotyledons such as tobacco, kalancho ë, and the like. However, it has been said that hosts of *Agrobacterium* are restricted to dicotyledons and that monocotyledons are not parasitized by the bacterium. Recently, transformation of gramineae plants, including rice, wheat, barely and maize which belong to monocotyledon by *Agrobacterium* has been reported (EP 0 672 752 A1 and EP 0 604 662 A1). These methods consist of transforming either dedifferentiated cultured tissue or non-dedifferentiated immature embryo of a gramineae plant with an *Agrobacterium* strain to provide a transgenic plant body. These methods provide good transforming efficiency, however, no one has shown that such methods as above can be applied to monocotyledons other than gramineae plants.

Orchidaceous plants belong to monocytledons and therefore they have been believed not to be hosts of *Agrobacterium*. In fact, our preliminary examination had shown that dedifferentiated cultured cells or non-dedifferentiated immature embryo of the orchidaceous plant failed to be infected with *Agrobacterium* (data not shown). Since regeneration of orchidaceous plant from protoplast is extremely difficult, the only method of transforming orchidaceous plants heretofore known in the art is to introduce the useful gene in epidermal of protocorm like body of an orchidaceous plant by using a particle gun (Japanese Patent Publication No. 07255300 A).

### DISCLOSURE OF THE INVENTION

An object of the present invention is to provide a method of generating a transgenic orchidaceous plant using *Agrobacterium*. Accordingly, the present invention provides a method of transforming an orchidaceous plant which comprises infecting a tissue derived from said orchidaceous plant with *Agrobacterium* containing a gene to be introduced into the genome of the orchidaceous plant. The present method enables the transformation of an orchidaceous plant with *Agrobacterium* for the first time.

In the present invention, the tissue derived from the orchidaceous plant is preferably a shoot derived from a node of the flower stalk of an orchidaceous plant.

According to the present invention, the infected tissue is preferably cultured to induce a protocorm like body (PLB), and then, a mature orchidaceous plant is regenerated from the PLB. According to the present method, homogeneously transformed mature orchidaceous plant can be obtained easily.

Another object of the present invention is to provide a transgenic orchidaceous plant that contains a desired gene. Accordingly, the present invention also provides a transgenic orchidaceous plant that is transformed with *Agrobacterium* according to the present method.

### DETAILED DESCRIPTION OF THE INVENTION

According to the present invention, the orchidaceous plant to be transformed by the present method may be any strain belonging to orcidaceae, including *Phalaenopsis*, *Doritaenopsis*, *Doritis*, *Paraphalaenopsis*, *Euphalaenopsis* and related genus thereof.

According to the present invention, the tissue to be infected with *Agrobacterium* may be a juvenile leaf differentiated from *in vitro* cultured protocorm or protocorm like body of the orchidaceous plant or a shoot sprouted from a node of a flower stalk of the orchidaceous plant. The shoot is most preferable. A method of inducing shoots from flower stalk cuttings is known in the art (M.Tanaka et., al. *Scientia Horticulture*, 35 (1988) 117-126). Briefly, flower stalk cuttings of an orchidaceous plant that are made by cutting the stalk around its nodes into about 1-3 cm segments may be cultured on an appropriate medium. Examples of the media preferably used to induce shoots from the flower stalk cuttings include Vacin-Went medium, Murashige-Skoog medium, Linsmaier-Skoog medium and the media which are the same as above but containing lower concentration of salts. These media can be supplemented with suitable agents known in the art. The most preferable medium is Vacin-Went medium consisting of 0.2g/l of Ca₃(PO₄)₂, 0.525g/l of KNO₃, 0.25g/l of KH₂PO₄, 0.5g/l of (NH₄)SO₄, 0.028g/l of iron(III) citrate n-hydrate, 0.0075g/l of MnSO₄ 4H₂O and 20g/l of sucrose.

The flower stalk cuttings may be incubated at about 20-30°C and about 50-90% RH. Usually, after about 1-3 months of incubation, the cuttings sprout shoots from their nodes. The obtained shoots are preferably used for infection with *Agrobacterium*.

*Agrobacterium* infection is known to occur at the wound site of a plant. Therefore, it is necessary to wound the plant and then to treat the wounded site with the bacterium. For example, in order to infect the shoot with *Agrobacterium*, the shoot may be wounded with a bamboo needle of which the point is coated with the bacterium.

*Agrobacterium* has been used as a vector for dicotyledon plant gene engineering. In the present specification, "*Agrobacterium*" represents a bacterium strain belonging to genus *Agrobacterium* or its derivatives. According to the present invention, *Agrobacterium* may be any strain known in the art for transformation of dicotyledon such as *Agrobacterium rhizogenes*, *Agrobacterium tumefaciens* or derivatives thereof. There are many commercially available Agrobacterium strains. In the present specification, the term "derivatives" represents mutants of the strain both naturally occurring and obtained by any method known in the art to introduce a desired gene or to enhance virulence of Ti or Ri plasmid contained in the bacterium.

When a plant is infected with *Agrobacterium*, T-DNA region of its Ti or Ri plasmid DNA is transferred and integrated into the genome of the plant cell. Usually, the T-DNA region of the plasmid contains the gene responsible for biosynthesis of phytohormone and therefore, expression of the T-DNA region in the infected host genome results in abnormal cell growth that causes tumor formation at the site of infection. In order to introduce a foreign gene into a plant host, the desired gene may be introduced in the T region of the Ti or Ri plasmid of the bacterium.

Methods of introducing a gene in the T-DNA region of the plasmid are well known in the art (Y. Hiei et. al., *The Plant Journal* (1994) 6(2) 271-282, EP 0 672-752 A1, EP 0 604 662 A1 and Y. NIWA in "Interaction between *Agrobacterium* and plant cells" *BISEIBUTSU* (1987) 3(4) 407-417). For example, a useful gene excised from a microorganism, plant or animal with an appropriate restriction enzyme, may be ligated in T-DNA region of the plasmid of *Agrobacterium* which is cleaved by the same restriction enzyme. Or, undesired gene in the said region can be removed by the same method as above. In addition, a commercially available strain, which contains a desired gene within said region could also be employed.

According to the present invention, the gene to be introduced into the orchidaceous plant may be any gene such as a gene responsible for disease resistance, viral resistance, insect resistance, dwarf formation, control of flower color and cold resistance.

According to the present invention, the T-DNA region of the plasmid preferably further contains a marker gene to select and confirm the transformant. Examples of the marker genes are well known in the art and include antibiotic resistant genes such as those resistant to kanamycin, G418, chloramphenicol and ampicillin. In addition, since Ti or Ri plasmid of *Agrobacterium* inherently contains a gene responsible for tumor formation at the infected site, tumor or swelling will be observed in the successfully infected plant. For example, when infected with *Agrobacterium rhizogenes*, tumor formation can be observed after about 3 weeks culture from the infection. Therefore, detecting swelling or tumor formation at the site treated with the bacterium can carry out 1st selection of successfully infected shoot.

The infected shoot is then cultured in a medium containing an antibiotic for additional 4 to 6 weeks to remove *Agrobacterium*. Example of antibiotic for removing *Agrobacterium* includes Claforan®, which is well known in the art.

Immediately after completion of the removal of the bacterium, the infected site, i.e. the swollen portion of the shoot is excised and cultured on protocorm like body-inducing medium to generate protocorm like body. Protocorm like body (PLB) of an orchidaceous plant is believed to be a tissue corresponding to an adventive embryo which is induced from explant such as shoot apex, leaf segment derived from flower stalk culture or root apex. And, it is believed that each PLB is derived from a single cell of the explant. Therefore, when a mature plant is regenerated from a transformed PLB, whole cells of the obtained plant may be homogeneously transformed.

Details for inducing PLB from a segment is reported by Michio TANAKA, Memoirs of Faculty of Agriculture, Kagawa University Vol. 49, pp 1-85 (1987). Briefly, the swollen portion of the shoot is excised and cut into segments about 1 cm square. The obtained segments are transplanted on a PLB inducing medium and cultured statically at about 25 °C, under light exposure (2000-3000 lux). An example of PLB inducing medium which is preferably used in the present invention consists of 3.5g/l of Hyponex® (N:P₂O₅:K₂O=7:6:19), 100 mg/l of myo-inositol, 1mg/l of Nicotinic acid, 1 mg/l of thiamine HCl, 1mg/l of α-naphtaleneacetic acid (NAA), 10 mg/l of N⁶-benzyladenine (BA), 10mg/l of adenine and 20g/l of sucrose. PLBs formation can be observed after about 100 days of culture.

The resulting PLBs are transplanted separately on to a differentiation medium to obtain juvenile plants. According to the present specification, "juvenile plant" represents a plant grown to the height of 1-2 cm. Differentiation medium for inducing a juvenile plant from PLB is known to the art, for example, a solid medium containing 3g/l of Hyponex®, 50g/l of potato extract and 20g/l sucrose is preferably used. PLB may be cultured under conventional culture condition known in the art.

When the *Agrobacterium* contains an antibiotic resistant gene in their T-DNA region, selection of transformant can be done before or after differentiation to juvenile plant from PLB. The selection can be carried out by culturing PLB or obtained juvenile plant on a selection medium containing said antibiotic.

In order to confirm whether the selected transformant contains the desired gene, there are some methods known in the art. For example, genomic DNA of the transformant may be amplified by using, for example, PCR technique and the amplified DNA may be analyzed to confirm the desired gene. When the infecting *Agrobacterium* contains a gene responsible for tumor formation in its T-DNA region, tumor formation on the juvenile plant regenerated from the transformed PLB can be a signal of transformation. In addition, the PLB or juvenile plant may be analyzed histologically to confirm expression of GUS gene.

The juvenile plant, which is confirmed as being successfully transformed may be grown up to adult plant by culturing in an appropriate medium. The culture condition may be the same as that of differentiation from PLB to juvenile plant.

### PREFERRED EMBODIMENT OF THE INVENTION

According to one embodiment of the present method, *Phalaenopsis*, an orchidaceous plant, is transformed with *Agrobacterium rhizogenes* R-1601, *Agrobacterium rhizogenes* 03-01724 and an *Agrobacterium rhizogenes* strain of ATCC No. 15834 respectively. These respective bacterium strains contain Ri plasmid having a rol C gene that is responsible for dwarfism within their T-DNA region. *Agrobacterium rhizogenes* R-1601 can be obtained from Professor Gordon, Department of Biochemistry, University of Washington, Seattle, WA 98195, U.S.A.; Agrobacterium rhizogenes 03-01724 can be obtained from National Institute of Agrobiological Resources, Japan.

Flower stalks of *Phalaenopsis* are cut around their nodes in cuttings of about 1-3 cm long. The cuttings are immersed in 1% aqueous benzalkonium chloride for 10 minutes and then in 1% effective chlorine concentration of aqueous hypochlorous acid for 20 minutes to sterilize the surface of the cuttings. The sterile cuttings are stuck on Vacin-Went medium consisting of 0.2g/l of Ca₃(PO₄)₂, 0.525g/l of KNO₃, 0.25g/l of KH₂PO₄, 0.028g/l iron(III) citrate n-hydrate, 0.0075g/l of MnSO₄4H₂O and 20g/l of sucrose and are cultured at 28°C, 90% RE for one month. After one month, shoots sprout from the nodes of the cuttings. Then the shoots are punctured with bamboo needles of which the points are coated with the above respective bacterium to be infected. The infected shoots are further cultured under the same condition.

Three weeks after the infection, the infected sites in the shoots are swollen. The swollen shoots, i.e. candidates for successfully infected shoots, are selected and further cultured on the medium containing 500 µg/ml of Claforan® for 30 days to remove the *Agrobacterium* from the shoot.

After completion of removing the bacterium, the swollen portions are excised and cut into about 1 cm square segments. Then, the segments are statically cultured on a PLB inducing medium consisting of 3.5g/l of Hyponex®, 100mg/l of myo-inositol, 1mg/l of nicotinic acid, 1mg/l of thiamine HCl, 1mg/l of NAA, 10mg/l of BA, 10mg/l of adenine and 20mg/l of sucrose (Michio TANAKA; Memoir of Faculty of Agriculture, Kagawa University vol.49, pp 1-85, 1987) at 25 °C under a light exposure of 2000 lux to induce PLB. The resulting PLBs are then cultured separately on a differentiation medium consisting of 3g/l of Hyponex®, 50g/l of potato extract and 20g/l of sucrose to induce juvenile plants.

The resulting juvenile plants are transplanted to a selection medium containing antibiotic to select the plant resistant to the antibiotic. The selected juvenile plants are further cultured under the same condition for inducing juvenile plants. When the T-DNA region of the *Agrobacterium* strain is successfully expressed in the juvenile plant, tumor is observed on the selected juvenile plant. After additional 3-5 months incubation under the same condition, mature dwarfism plants and mature sympodial plants are obtained.

To further illustrate this invention, and not by way of limitation, the following examples are given.

### EXAMPLE 1

Flower stalk of *Phalaenopsis* was cut around their nodes into cuttings of about 1-3 cm long. The cuttings were immersed in 1% aqueous benzalkonium chloride for 10 minutes and then in 1% effective chlorine concentration of aqueous hypochlorous acid for 20 minutes to sterilize surface of the cuttings. The sterile cuttings were stuck on Vacin-Went medium consisting of 0.2g/l of Ca₃(PO₄)₂, 0.525g/l of KNO₃, 0.25g/l of KH₂PO₄, 0.028g/l iron(III) citrate n-hydrate, 0.0075g/l of MnSO₄4H₂O and 20g/l of sucrose, and cultured at 28°C, 90% RH for one month.

After one month's culture, shoots sprouted from the nodes of the cuttings. Then the shoots were infected with *Agrobacterium rhizogenes* R-1601 and cultured on the same medium. The bacterium strain used here was purchased from Prof. Milton P. Gordon of Department of Biochemistry, University of Washington, Seattle, WA 98195, U.S.A.. The nature of the bacterium is described by Pythoud et al., Bio/Technology, 5, 1323-1327 (1987). The Ri plasmid of the strain contains special vir region, which increases virulence of the bacterium. In addition, the plasmid contains G418 resistant region and rol C gene that is responsible for dwarfism within its T-DNA region.

Three months after infection, the infected sites in the shoots were swollen. The swollen shoots were selected and further cultured for 30 days on a medium containing 500 µg/ml of Claforan® to remove *Agrobacterium* from the shoot.

After completion of removing the bacterium, the swollen portions were excised and cut into about 1 cm square segments. Then, the segments were statically cultured on a PLB inducing medium consisting of 3.5g/l of Hyponex®, 100mg/l of myo-inositol, 1mg/l of nicotinic acid, 1mg/l of thiamine HCl, 1mg/l of NAA, 10mg/l of BA, 10mg/l of adenine and 20g/l of sucrose at 25 °C under light exposure of 2000 lux to generate PLB. The resulting PLBs were then cultured separately on a differentiation medium consisting of 3g/l of Hyponex®, 50g/l of potato extract and 20g/l of sucrose to induce juvenile plants.

The resulting juvenile plants were transplanted to a medium containing 20 µg/ml of G418 to select a plant resistant to the antibiotic. The selected juvenile plants were further cultured under the same condition for inducing juvenile plants. Tumors observed on the selected juvenile plant showed that T-DNA regions of *Agrobacterium* were successfully expressed in the regenerated plants. The selected juvenile plants were cultured for additional 3-5 months under the same condition. At the consequence, mature dwarfism plants and mature sympodial plants were obtained.

### INDUSTRIAL APPLICABILITY

The present invention provides a method of transforming an orchidaceous plant with *Agrobacterium* for the first time. According to the present invention, one can obtain transgenic orchidaceous plant which contains a desired gene easily.

## Claims

1. A method of transforming an orchidaceous plant comprising the step of;
infecting a tissue derived from said orchidaceous plant with *Agrobacterium* having a gene to be introduced into the genome of the plant.

2. The method of claim 1, wherein the tissue derived from said orchidaceous plant is a shoot sprouted from a node of flower stalk of the orchidaceous plant.

3. The method of claim 1, further comprising the step of;
inducing a protocorm like body from the infected tissue and then differentiating said protocorm like body into adult plant.

4. The method of claim 1, wherein the *Agrobacterium* is selected from the group consisting of *Agrobacterium rhizogenes*, *Agrobacterium tumefaciens* and derivatives thereof.

5. The method of claim 4, wherein the *Agrobacterium* is *Agrobacterium rhizogenes* or its derivative.

6. The method of claim 1, wherein the orchidaceous plant is selected from the group consisting of *Phalaenopsis*, *Doritaenopsis*, *Doritis*, *Paraphalaenopsis*, *Euphalaenopsis* and related genus thereof.

7. The method of claim 6, wherein the orchidaceous plant is *Phalaenopsis*.

8. A transformed orchidaceous plant obtained by transforming an orchidaceous plant by a method comprising the step of infecting a tissue derived from said orchidaceous plant with *Agrobacterium* having a gene to be introduced into the genome of the plant.
